(19) **Europäisches Patentamt**

**European Patent Office**

**Office européen des brevets**

(11) **EP 0 658 099 B1**

(12) **FASCICULE DE BREVET EUROPEEN**

(45) Date de publication et mention
de la délivrance du brevet:
**13.08.1997 Bulletin 1997/33**

(21) Numéro de dépôt: **92910922.1**

(22) Date de dépôt: **30.04.1992**

(51) Int. Cl.$^6$: **A61K 7/48**, A61K 38/44

(86) Numéro de dépôt international:
**PCT/FR92/00400**

(87) Numéro de publication internationale:
**WO 92/19224 (12.11.1992 Gazette 1992/28)**

(54) **COMPOSITION TOPIQUE ANTI RADICAUX LIBRES A BASE DE SUPEROXYDE-DISMUTASE ET D'UN DERIVE PHOSPHONIQUE**

TOPISCHES MITTEL GEGEN FREIE RADIKALE, DAS SUPEROXYD-DISMUTASE UND EIN PHOSPHONSÄUREDERIVAT ENTHÄLT

ANTI-FREE-RADICAL TOPICAL COMPOSITION BASED ON A SUPEROXIDE DISMUTASE AND A PHOSPHONIC DERIVATIVE

(84) Etats contractants désignés:
**AT BE CH DE DK ES FR GB GR IT LI MC NL SE**

(30) Priorité: **03.05.1991 FR 9105464**

(43) Date de publication de la demande:
**21.06.1995 Bulletin 1995/25**

(73) Titulaire: **L'OREAL**
**75008 Paris (FR)**

(72) Inventeurs:
• **N'GUYEN, Quang Lan**
**F-92160 Antony (FR)**
• **GALEY, Jean-Baptiste**
**F-75005 Paris (FR)**

(74) Mandataire: **Stalla-Bourdillon, Bernard**
**CABINET NONY & CIE**
**29, rue Cambacérès**
**75008 Paris (FR)**

(56) Documents cités:
**EP-A- 0 193 925          EP-A- 0 273 579**
**US-A- 4 784 790**

**Description**

La présente invention a pour objet des compositions cosmétiques ou pharmaceutiques contenant une superoxyde-dismutase (SOD) en association avec les dérivés d'acide phosphonique en tant qu'agent complexant des métaux.

De telles compositions trouvent application par administration topique notamment dans le lutte contre le vieillissement cutané et dans la protection de la peau des irradiations.

On sait que les superoxyde-dismutases sont des enzymes capables d'induire la dismutation des ions superoxydes, suivant la réaction :

$$2\ O_2 + 2\ H^+ \xrightarrow{\text{SOD}} H_2O_2 + O_2$$

On a déjà décrit des superoxyde-dismutases extraites d'érythrocytes de boeuf (Markovitz, J. Biol. Chem. 234, p 40, 1959) et des superoxyde-dismutases extraites d'Escherichia coli (Keele et Fridovich, J. Biol. Chem., 245 , p 6176, 1970).

Dans la demande de brevet français n° 73.13670 déposée le 16 Avril 1973 sont décrites des superoxyde-dismutases extraites de souches bactériennes marines, ainsi que leur procédé de préparation.

Les superoxyde-dismutases permettent notamment de protéger la peau et les cheveux en maintenant l'intégrité de la structure kératinique naturelle tel que décrit par exemple dans la demande de brevet français n° 75.31354. Il est possible que cette protection soit due à l'inhibition de phénomènes d'oxydation de la kératine. Les superoxyde-dismutases améliorent la respiration cellulaire cutanée et maintiennent ou améliorent les qualités de la peau : douceur au toucher, souplesse et élasticité. Leur présence dans des compositions pour cheveux permet aussi le maintien ou l'amélioration de l'état du cuir chevelu, tout en protégeant également la peau des mains de la personne qui applique ces compositions.

En outre, les superoxyde-dismutases protègent la peau contre les phénomènes d'inflammation causés par les rayonnements ultra-violets ainsi que contre le vieillissement de la peau dû à ce même stimulus.

Grâce à ces différentes propriétés, les superoxyde-dismutases sont utilisables dans des compositions cosmétiques pour la peau ou pour les cheveux ainsi que dans des compositions pharmaceutiques à usage dermatologique ou ophtalmologique.

En effet, l'ion superoxyde $O_2^{\dot{-}}$ (oxygène actif) est un radical libre dont l'instabilité et la réactivité dans le cas d'un organisme en font un composé toxique car il engendre, en présence d'ions métalliques, des radicaux hydroxyle (OH ) hautement toxiques.

La SOD exerce un effet protecteur en piègeant les ions superoxydes et constitue donc un système biologique de défense contre les effets toxiques de l'oxygène.

Il a été mis en évidence que les agents complexants et donc inactivateurs de métaux peuvent dans certains cas atténuer la production des radicaux OH toxiques.

Or, il a maintenant été constaté de manière tout à fait inattendue et surprenante que parmi les agents complexants de métaux, tous ne conviennent pas car certains d'entre eux sont en outre activateurs de réactions radicalaires agissant ainsi à l'encontre d'une suppression de la formation de tels radicaux libres.

La présente invention a donc pour objet une composition anti-radicalaire caractérisée par le fait qu'elle comprend la superoxyde-dismutase en association avec au moins un parmi une sélection particulière d'agents complexants de métaux, nommément les dérivés de l'acide phosphonique, de préférence les dérivés polyphosphoniques, qui ne stimulent pas en même temps la formation de radicaux libres. Au contraire, ces dérivés renforcent d'une manière synergique l'action protectrice exercée par la SOD.

L'invention a tout particulièrement pour objet une composition cosmétique destinée à un usage topique caractérisée par le fait qu'elle comprend au moins une superoxyde-dismutase en association avec au moins un dérivé phosphonique en tant que complexant de métaux.

L' effet de synergie ci-dessus mentionné entre la SOD et le composé phosphonique est particulièrement marqué lorsque le rapport pondéral de SOD au(x) composé(s) phosphonique(s) est compris entre 2 et 25 et de préférence entre 10 et 25.

Par l'expression "SOD" on entend tout composant à activité superoxyde-dumutase à savoir tout enzyme qui peut catalyser la réaction de dismutation ci-dessus indiquée ainsi que tout autre produit ayant cette activité ce qui inclut notamment les SOD modifiées par greffage de polyalkylène oxyde, polyéthylèneglycol, polysaccharide ou groupes acylés ainsi que les substances contenant de tels produits. On peut citer à cet égard la demande de brevet européen n° EP 223.257.

La SOD utilisée selon l'invention peut ainsi être celle modifiée notamment selon l'enseignement extrait de "International Conference on Medical, Biochemical and Chemical Aspects of Free Radicals" (Avril p.9-13, 1988 Kyoto) p. 317 dans l'article de MORIMOTO H., ou selon l'enseignement de M. YUKIO Ando p.318 (même source), ou encore d'après JP 01250304 (Kanebo).

La SOD utilisée selon l'invention peut être en outre employée sous forme stabilisée à l'aide de techniques connues par exemple au phosphate, en présence de chlorure de métal alcalin et de saccharose tels que publiées par exemple dans FR 2.634.125 (Nippon).

Toutes les superoxyde-dismustases ci-dessus décrites ainsi que les variantes et équivalentes que l'homme du métier peut déduire de cette littérature conviennent en tant que SOD selon l'invention.

Les SOD utilisables selon l'invention peuvent être d'origine diverse.

On peut notamment citer les SOD d'origine animale (par exemple bovine, porcine,....), humaine (par exemple placenta, sang,.....), bactérienne, levures, végétales (par exemple champignons, algues, épinards,......) ou biotechnologique (par exemple manipulation génétique.....).

Parmi les exemples de SOD d'origine bovine on peut citer en particulier la SOD de type Cu-Zn qui a été purifiée jusqu'à homogénéité et approuvée pour des applications cliniques (New Trends in Allergy, I. Ring et al, Ed. Springer Verlag 1986).

Parmi les exemples de la SOD obtenue par voie biotechnologique à partir notamment de cultures de bactéries, levures, cellules animales, etc., on peut citer la SOD Cu-Zn humaine recombinante de la Société UBE Industries Ltd.

Parmi les exemples de SOD extraites de bactéries, on peut citer en particulier celles extraites d'Escherichia coli ; parmi les superoxyde-dismutases extraites de champignons, on peut citer en particulier celles extraites de Pleurotus olearius ; parmi les superoxyde-dismutases extraites du sang, on peut citer les érythrocupréines.

On peut citer également les superoxyde-dismutases extraites de souches bactériennes marines, telles que par exemple des souches de Photobactérium phosphoreum, Photobactérium leiognathi ou Photobactérium sépia.

Parmi les diverses souches utilisables, on peut citer les souches de Photobactérium phosphoreum n° ATCC 11040, de Photobactérium leiognathi n° ATCC 25521, de Photobactérium sépia n° ATCC 15709, d'Escherichia coli n° ATCC 15224 et de Pleurotus olearius Gillet (Laboratoire de Cryptogamie de Paris).

Les SOD utilisées selon l'invention peuvent être préparées par application des méthodes déjà décrites par exemple dans l'article de KEELE et al (supra) ainsi que dans Eur. J. Rheumatol. and Inflammation, 4, 173-182 (1982).

Dans le cas des superoxyde-dismutases extraites de souches bactériennes marines, celles-ci peuvent être préparées selon le procédé décrit dans la demande de brevet français n° 73.13670 ci-dessus mentionnée.

Il existe plusieurs types de SOD utilisables selon l'invention, à savoir les SOD à fer, à manganèse ou à cuivre-zinc, cette dernière étant préférée.

Les agents complexant de métaux utilisables selon l'invention sont des composés présentant dans la molécule au moins une fonction phosphonique et de préférence ceux en présentant plusieurs.

De tels dérivés polyphosphoniques sont de préférence choisis parmi l'acide éthylènediamine tétra(méthylène phosphonique), l'acide hexaméthylène diamine tétra(méthylène phosphonique), l'acide diéthylènetriamine penta (méthylène phosphonique), l'acide 1-hydroxy-éthylidène 1,1-diphosphonique, l'acide aminotri(méthylène phosphonique) ainsi que leur sels notamment ceux d'ammonium ou de métaux alcalins.

Les dérivés polyphosphoniques utilisables d'une manière particulièrement préférentielle selon l'invention sont en particulier l'acide éthylènediamine tétra (méthylène phosphonique), l'acide diéthylène triamine penta (méthylène phosphonique) et leur sel de sodium. On peut notamment employer ces composés tels que commercialisés sous les dénominations de DEQUEST 2041®, 2046®, 2061® et 2066® par la Société MONSANTO.

L'association binaire antiradicaux libres selon l'invention trouvant principalement une application dans des compositions cosmétiques destinées à lutter contre le vieillissement cutané et à protéger la peau ou les cheveux des irradiations, l'invention a donc également pour objet de telles compositions cosmétiques ou dermatologiques.

Dans les compositions topiques selon l'invention, la SOD représente généralement de 0,001 à 4 % en poids, de préférence de 0,75 à 1,7 % ; et le dérivé d'acide phosphonique est généralement présent à raison de 0,005 % à 2 % en poids, de préférence de 0,05 % à 0,1 en poids.

Les compositions selon l'invention destinées à une application topique sont notamment des solutions ou dispersions du type lotion ou sérum, des émulsions de consistance liquide ou semi-liquide du type lait, obtenues par dispersion d'une phase grasse dans une phase aqueuse (H/E) ou inversement (E/H), ou des suspensions ou émulsions de consistance molle du type crème ou gel, ou encore des microsphères, microgranulés, ou des dispersions vésiculaires de types ioniques et/ou non ioniques.

Ces compositions sont préparées selon les méthodes usuelles. Elles constituent notamment des crèmes de nettoyage, de protection ou de soin pour le visage, pour les mains ou pour le corps, (par exemple crèmes de jour, crèmes de nuit, crèmes démaquillantes, crèmes fond de teint, crèmes anti-solaires), des fonds de teint fluides, des laits de démaquillage, des laits corporels de protection ou de soin, des laits anti-solaires ; des lotions de nettoyage, des lotions anti-solaires, des lotions de bronzage artificiel, des compositions pour bain, ou des compositions désodorisantes contenant un agent bactéricide.

Les compositions selon l'invention peuvent également consister en des préparations solides constituant des savons ou des pains de nettoyage.

Les compositions qui sont de nature fluide peuvent être présentées sous forme de bombe aérosol contenant également un propulseur sous pression.

Les compositions pour la peau selon l'invention contiennent, outre l'association binaire selon l'invention des ingrédients actifs ou excipients utilisés de façon usuelle dans les formulations mentionnées ci-dessus, tels que des tensio-actifs, des colorants, des parfums, des agents conservateurs, des émulsionnants, des véhicules liquides tels que l'eau, des corps gras tels que des huiles naturelles ou synthétiques destinées à constituer la phase grasse des laits ou des crèmes, des résines, etc..., Les composés destinés à constituer une phase grasse sont par exemple, des huiles minérales, animales, végétales ou synthétiques, des cires, des alcools gras ou encore des acides gras.

Parmi les huiles minérales, on peut citer par exemple l'huile de vaseline, et parmi les huiles synthétiques, les palmitates d'éthyle et d'isopropyle, les myristates d'alkyle tels que le myristate d'isopropyle, de butyle, de cétyle, le stéarate d'hexyle, les triglycérides des acides octanoïque et décanoïque (par exemple le produit vendu sous la dénomination de "MIGLYOL" par la Société DYNAMIT-NOBEL), le ricinoléate de cétyle, l'octanoate de stéaryle (huile de purcellin) et de polyisobutène hydroxylé.

Parmi les huiles végétales, on peut citer par exemple l'huile d'amande douce, l'huile d'avocat, l'huile de coco, l'huile de germe de blé, l'huile de maïs, l'huile de ricin, l'huile d'olive, l'huile de palme, l'huile de sésame, l'huile de soja, l'huile d'argan, l'huile d'onagre, l'huile de bourrache, les huiles essentielles et les cires végétales telles que la cire d'abeille ou encore les cires synthétiques.

Parmi les alcools gras, on peut citer l'alcool cétylique, l'alcool stéarylique, l'alcool myristique, l'alcool hydroxystéarylique, l'alcool oléique, l'alcool isostéarylique, l'alcool laurylique, l'alcool hexadécylique, l'alcool ricinoleylique, l'alcool béhénylique, l'alcool érucylique et le 2-octyldodecanol.

Parmi les acides gras, on peut mentionner l'acide stéarique, l'acide myristique, l'acide palmitique, l'acide oléique, l'acide linoléique, l'acide laurique, l'acide isostéarique, l'acide hydroxystéarique, l'acide linolénique, l'acide ricinoléique, l'acide arachidique, l'acide béhénique, l'acide érucique et les acides lanoliniques.

Les compositions pour cheveux selon l'invention peuvent être présentées sous forme de solutions aqueuses, alcooliques ou hydroalcooliques, ou sous forme de crèmes, de gels, d'émulsions, de mousses ou encore sous forme de bombes aérosols contenant également un agent propulseur sous pression.

Outre les ingrédients actifs classiques, elles peuvent renfermer divers adjuvants habituellement présents dans ces compositions pour cheveux par exemple des véhicules liquides ou sous forme de gels, des parfums, des colorants, des agents conservateurs, des agents épaississants, etc.

Elles constituent par exemple des crèmes, lotions, gels, sérums ou mousses pour le soin de la peau, des shampooings, des lotions de mise en plis, des lotions traitantes, des crèmes ou des gels coiffants, des compositions de teintures (notamment teintures d'oxydation) éventuellement sous forme de shampooings colorants, des lotions restructurantes, des compositions de permanente (notamment des compositions pour le premier temps d'une permanente), des lotions ou des gels antichute, etc.....

Les compositions de l'invention sont par exemple :

- des shampooings contenant, outre une superoxyde dismutase et le dérivé polyphosphonique, un détergent cationique, anionique ou non ionique,
- des compositions de teinture y compris des shampooings colorants, qui contiennent des colorants ou des précurseurs de colorant tels que ceux déjà mentionnés précédemment par exemple sulfate de m-diaminoanisole, o-, m-ou p-aminophénol, nitroparaphénylènediamine, paraphénylène-diamine, p-toluènediamine, 5,6-dihydroxy indole, etc...;
- des compositions pour le premier temps (temps de réduction) d'une déformation permanente des cheveux, contenant des dérivés réducteurs tels que mercaptans, sulfites, etc...
- des compositions pour le ralentissement de la chute des cheveux et pour favoriser la repousse des cheveux, contenant des composés tels que le "Minoxidil" (2,4-diamino-6-pipéridino-pyrimidine-3-oxyde) et ses dérivés, le Diazoxide (7-chloro-3-méthyl-1,2,4-benzothiadiazine 1,1-dioxyde) et le "Phenytoïn" (5,5-diphényl imidazolidine 2,4-dione).

Il convient de remarquer que les compositions cosmétiques selon l'invention sont aussi bien des compositions prêtes à l'emploi que des concentrés devant être dilués avant l'utilisation. Les compositions pouvant être présentées sous forme de concentrés sont par exemple des shampooings ou des compositions pour bains.

Les compositions selon l'invention renferment la SOD et le dérivé phosphonique soit à titre d'ingrédient actif principal, soit à titre de protecteur contre l'oxydation des autres ingrédients.

Dans le cas où l'ingrédient oxydable à protéger subit une décomposition accélérée en présence des fibres kératiniques et/ou de la peau, la SOD avec le dérivé phosphonique peuvent être conservés seuls, en solution aqueuse diluée ou concentrée, ou sous forme de complexe ou de lyophilisat, et être ajoutés aux autres ingrédients de la composition au moment de l'emploi.

De même, lorsque la SOD et le dérivé phosphonique sont utilisés dans le but de maintenir ou d'améliorer les qualités de la peau ou des cheveux, ces substances peuvent n'être ajoutées à la composition qu'au moment de l'emploi.

Les compositions selon l'invention peuvent donc se présenter sous la forme d'un conditionnement en plusieurs parties contenant d'une part la SOD avec le dérivé phosphonique, et d'autre part les autres ingrédients de la composition.

Comme indiqué ci-dessus, la SOD et le dérivé phosphonique peuvent être conservés par exemple sous forme de solution aqueuse, de complexe ou de lyophilisat.

La présente invention a en outre pour objet un traitement cosmétique caractérisé par le fait que l'on applique sur les cheveux ou sur la peau une composition contenant au moins une SOD en association avec au moins un dérivé phosphonique.

Le procédé de traitement cosmétique de l'invention peut être mis en oeuvre par application des compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou les cheveux, application d'une lotion pour cheveux sur cheveux mouillés, shampooings...

Le procédé de traitement cosmétique de l'invention est mis en oeuvre de façon à appliquer une quantité efficace de SOD et de dérivé phosphonique, c'est-à-dire une quantité suffisante, pour obtenir l'effet de protection recherché.

Ce procédé de traitement cosmétique est destiné soit à maintenir la structure kératinique de la peau ou des cheveux, soit à maintenir ou améliorer les qualités de la peau (douceur, souplesse, élasticité), soit à protéger la peau contre les effets nocifs des rayons ultra-violets.

<u>ETUDE D'ACTIVITE</u>

La potentialisation des propriétés de la SOD par les dérivés d'acide phosphonique selon l'invention a été comparée à un autre type d'agent complexant de métaux, à savoir l'EDTA (l'acide éthylène diamine tétracétique). Elle a été mise en évidence in vitro, par une méthode d'évaluation du piégeage des radicaux libres basée sur la mesure de la formation d'éthylène.

Cette méthode consiste à mesurer par chromatographie en phase gazeuse, l'éthylène formé à partir de l'oxydation de la méthionine par le radical hydroxyle.

Le mode opératoire consiste à mélanger, dans un flacon "head space", l'actif à étudier avec le "stress" oxydatif (acide ascorbique et sulfate de cuivre) qui génère les espèces réduites de l'oxygène ($O_2$, $H_2O_2$, OH) et avec le marqueur, la méthionine. Le milieu est incubé pendant 1 heure à 37°C. Puis la mesure de la quantité d'éthylène générée est évaluée en chromatographie en phase gazeuse.

L'EDTA a été étudié seul et associé avec la SOD de la même manière qu'un dérivé phosphonique selon l'invention, à savoir l'acide éthylènediamine tétra (méthylène phosphonique) appelé ci-après DEQUEST 2041[®] selon la dénomination commerciale de la Société MONSANTO.

L'éthylène ainsi formé est alors quantifié en chromatographie en phase gazeuse, par prélèvement d'échantillons d'air à l'intérieur de la cellule. La hauteur du pic de chromatogramme obtenu correspond donc à la quantité d'éthylène générée, la hauteur maximum obtenue en l'absence de l'actif représentant une inhibition de 0 %.

Les résultats pour le DEQUEST 2041[®] sont donnés dans le tableau 1 et ceux pour l'EDTA dans le tableau 2.

TABLEAU 1

| (DEQUEST[®]) | | |
|---|---|---|
| SOD mg/ml | DEQUEST 2041[®] (mg/ml d'une solution à 88%) | POURCENTAGE INHIBITION radicalaire |
| - | - | 0 |
| 0,05 | - | 35,6 |
| - | 0,0035 | 20,8 |
| 0,025 | 0,00175 | 57,9 |

On remarque donc que l'utilisation simultanée de SOD et de DEQUEST 2041[®] à une concentration en matière active totale largement inférieure à 0,05 mg/ml permet une inhibition radicalaire beaucoup plus importante que celle obtenue pour la SOD seule à une concentration de 0,05 mg/ml ce qui démontre une inhibition synergique de la génération des radicaux libres.

TABLEAU 2

| (EDTA) | | |
|---|---|---|
| SOD mg/ml | EDTA (mg/ml d'une solution à 88%) | POURCENTAGE INHIBITION radicalaire |
| - | - | 0 |
| 0,05 | - | 35,6 |
| - | 0,0035 | -0,04 |
| 0,025 | 0,00175 | 7,0 |

On remarque donc que l'utilisation simultanée de SOD et de l'EDTA provoque une inhibition de 7 % de la génération des radicaux libres. Cette inhibition est donc inférieure à celle obtenue pour la SOD seule ce qui ne démontre pas l'effet de synergie.

On va maintenant donner à titre d'illustration plusieurs exemples de compositions cosmétiques selon l'invention.

| EXEMPLE 1 : Emulsion H/E | |
|---|---|
| | % en poids |
| SOD Cu-Zn (M.A.) | 0,08 |
| Acide éthylènediamine tétra(méthylènephosphonique)(DEQUEST 2041®) (M.A.) | 0,005 (11µmoles) |
| Polyéthylène glycol 50 oxyéthyléné | 1,5 |
| Stéarate de monodiglycéryle | 1,5 |
| Huile de vaseline | 24 |
| Alcool cétylique | 2,5 |
| Triéthanolamine q.s. pH = 7 | |
| Eau q.s.p. | 100 |

| EXEMPLE 2 : Emulsion E/H | |
|---|---|
| | % en poids |
| SOD-Mn          (M.A.) | 0,026 |
| Sel pentasodique de l'acide éthylènediamine tétra (méthylène phosphonique)(DEQUEST 2046®) (M.A.) | 0,013 (24μmoles) |
| Sesquiisostéarate de polyglycéryle | 4,0 |
| Cire d'abeille blanche | 0,5 |
| Stéarate de magnésium | 1,5 |
| Stéarate d'aluminium | 1 |
| Huile de ricin hydrogéné oxyéthyléné à l'aide de 7 moles d'oxyde d'éthylène | 3 |
| Palmitate d'isopropyle | 10 |
| Perhydrosqualène | 15 |
| Eau q.s.p. SOD Cu-Zn          (M.A.) | 100 1 |
| EXEMPLE 3 : Emulsion H/E | |
| SOD Cu-Zn          (M.A.) | 1 |
| Sel heptasodique de l'acide diéthylène triamine penta (méthylène phosphonique)(DEQUEST 2066®)(M.A.) | 0,1 (50μmoles) |
| Ether cétéarylique du polyéthylène glycol | 5 |
| Alcool cétylique | 1 |
| Stéarate de glycéryle | 1 |
| Huile de vaseline | 6 |
| Myristate d'isopropyle | 3 |
| Dimethicone | 1 |
| Glycérine | 5 |
| Parahydroxybenzoate de méthyle | 0,3 |
| Eau qsp | 100 |

| EXEMPLE 4 : Sérum | |
|---|---|
| | % en poids |
| Lécithine de soja Epikuron 200® (vendue par la Société Lucas MEYER) | 0,75 |
| Cholestérol | 0,20 |
| Acylglutamate de sodium HS21® (vendue par la Société AJINOMOTO) | 0,05 |
| Glycérine | 1 |
| SOD Cu-Zn        (M.A.) | 0,04 |
| Acide éthylènediamine tétra-(méthylène phosphonique)(DEQUEST 2041®) | 0,008 (18µmoles) |
| Parahydroxybenzoate de méthyle | 0,2 |
| Mélange d'acides carboxyvinyliques "CARBOPOL 940®"(vendu par GOODRICH) | 0,1 |
| Triéthanolamine        qs pH = 7 | |
| Eau qsp | 100 |

EXEMPLE 5 : Dispersion vésiculaire

| | % en poids |
|---|---|
| Amphiphile non ionique *..................... | 0,9 |
| Acylglutamate de sodium HS21® (vendue par la Société AJINOMOTO).......................... | 0,1 |
| Glycérine.................................... | 3,00 |
| SOD Cu-Zn...................................(M.A.) | 0,08 |
| Acide éthylènediamine tétra-(méthylène phosphonique)(DEQUEST 2041®)................. | 0,005 (11µmoles) |
| Perhydrosqualene........................... | 10 |
| Parahydroxybenzoate de méthyle.............. | 0,2 |
| Mélange d'acides carboxyvinyliques "CARBOPOL 940®"(vendu par GOODRICH)......... | 0,4 |
| Triéthanolamine...................qs pH = 7 | |
| Eau qsp.................................... | 100 |

(*) L'amphiphile non ionique a la formule suivante :

$$C_{12}H_{25}\left[OC_2H_3(R)-O-C_3H_5(OH)-O\right]_n H$$

où $-OC_2H_3(R)-$ est constitué par un mélange des radicaux :

$$-O-\underset{R}{CH}-CH_2- \quad et \quad -O-CH_2-\underset{R}{CH}- \; ;$$

où $-C_3H_5(OH)-O-$ est constitué par un mélange de radicaux :

$$CH_2-\underset{CH_2OH}{CH}-O- \quad et \quad -\underset{CH_2OH}{CH}-CH_2-O- \; ;$$

où $n = 6$ ;

et où R est un mélange des radicaux $C_{14}H_{29}$ et $C_{16}H_{33}$.

| EXEMPLE 6 : Gel anti-chute | |
|---|---|
| | % en poids |
| SOD Cu-Zn        (M.A.) | 0,05 |
| Sel pentasodique de l'acide éthylènediamine tétra-(méthylène phosphonique)(DEQUEST 2046®) (M.A.) | 0,07 (129µmoles) |
| Propylène glycol | 5 |
| "CARBOPOL 934®"(mélange d'acides carboxyvinyliques) | 0,5 |
| Minoxidil (supra) | 1 |
| Triéthanolamine        qs pH = 7 | |
| Conservateurs q.s. | |
| Eau qsp | 100 |

| EXEMPLE 7 : Lait solaire | |
|---|---|
| | % en poids |
| Alcool cétylstéarylique | 2,6 |
| Alcool cétylstéarylique oxyéthylène 330E | 0,6 |
| Huile de vaseline | 6 |
| Myristate d'isopropyle | 3 |
| Alcool stéarylique | 2,5 |
| Ditertiobutyl-4-hydroxytoluène | 0,025 |
| Para-méthyl benzylidène camphre | 2,5 |
| P-méthoxycinnamate de 2-éthylhexyle | 4 |
| Glycérol | 1,2 |
| SOD Cu-Zn | 0,2 |
| Sel pentasodique de l'acide éthylènediamine tétra-(méthylène phosphonique)(DEQUEST 2046®) (M.A.) | 0,015 (28µmoles) |
| Conservateurs qs | |
| Parfums qs | |
| Eau qsp | 100 |

## Revendications

1. Composition cosmétique ou pharmaceutique destinée à un usage topique caractérisée par le fait qu'elle comprend au moins une superoxyde-dismutase en association avec au moins un dérivé d'acide phosphonique ou l'un de ses sels.

2. Composition selon la revendication 1, caractérisée par le fait que la superoxyde-dismutase et le dérivé phosphonique sont présents dans un rapport pondéral compris entre 2 et 25 %.

3. Composition selon la revendication 1 ou 2, caractérisée par le fait que la superoxyde-dismutase est présente à raison de 0,001 à 4 % en poids et de préférence de 0,75 à 1,7 % en poids par rapport au poids total de la composition.

4. Composition selon l'une quelconque des revendications 1 à 3, caractérisée par le fait que le dérivé phosphonique est présent à raison de 0,005 à 2 % en poids et de préférence de 0,05 à 0,1 % en poids par rapport au poids total de la composition.

5. Composition selon l'une quelconque des revendications 1 à 4, caractérisée par le fait que la superoxyde-dismutase est d'origine animale, humaine, bactérienne, levure, végétale, marine ou obtenue par biotechnologie.

6. Composition selon l'une quelconque des revendications 1 à 5, caractérisée par le fait que la superoxyde-dismutase est à fer, à manganèse ou à cuivre-zinc.

7. Composition selon l'une quelconque des revendications 1 à 6, caractérisée par le fait que le dérivé polyphosphonique est choisi parmi l'acide éthylénediamine tétra (méthylène phosphonique), l'acide hexaméthylènediamine tétra(méthylène phosphonique), l'acide diéthylènetriamine penta (méthylènephosphonique), l'acide 1-hydroxyéthylidène 1,1-diphosphonique, et l'acide aminotri(méthylène phosphonique) et de préférence parmi l'acide éthylènediamine tétra(méthylène phosphonique), et l'acide diéthylènetriamine penta (méthylène phosphonique) ainsi que leur sels.

8. Composition selon l'une quelconque des revendications 1 à 7, caractérisée par le fait que les sels sont choisis parmi ceux d'ammonium et de métaux alcalins, de préférence de sodium.

9. Composition selon l'une quelconque des revendications 1 à 8, caractérisée par le fait qu'elle est sous forme d'une lotion, d'une émulsion, d'un lait, de microsphères, de microgranulés, d'une crème, d'un gel, d'un baume ou d'une bombe aérosol, ou de vésicules de type ionique ou non ionique.

10. Composition selon l'une quelconque des revendications 1 à 9, caractérisée par le fait qu'elle contient en outre au moins un ingrédient cosmétique choisi parmi des tensio-actifs, des colorants, des parfums, des agents conservateurs, des émulsionnants, des véhicules liquides, des corps gras, des résines et des cires.

11. Utilisation d'au moins une superoxyde-dismutase en association avec au moins un dérivé phsponique, de préférence choisi parmi l'acide éthylénediamine tétra (méthylène phosphonique), l'acide hexaméthylènediamine tétra(méthylène phosphonique), l'acide diéthylènetriamine penta (méthylènephosphonique), l'acide 1-hydroxyéthylidène 1,1-diphosphonique et l'acide aminotri(méthylène phosphonique) ainsi que leurs sels dans la préparation d'une composition cosmétique destinée au traitement de la peau.

12. Procédé de traitement cosmétique en vue d'améliorer l'aspect esthétique de la peau, caractérisé par le fait qu'on apporte sur la peau une composition cosmétique selon les revendications 1 à 10.

## Claims

1. Cosmetic or pharmaceutical composition intended for a topical use, characterized in that it comprises at least one superoxide dismutase in combination with at least one phosphonic acid derivative or one of its salts.

2. Composition according to Claim 1, characterized in that the superoxide dismutase and the phosphonic derivative are present in a ratio by weight of between 2 and 25 %.

3. Composition according to Claim 1 or 2, characterized in that the superoxide dismutase is present in the proportion of 0.001 to 4 % by weight and preferably of 0.75 to 1.7 % by weight with respect to the total weight of the composition.

4. Composition according to any one of Claims 1 to 3, characterized in that the phosphonic derivative is present in the proportion of 0.005 to 2 % by weight and preferably of 0.05 to 0.1 % by weight with respect to the total weight of the composition.

5. Composition according to any one of Claims 1 to 4, characterized in that the superoxide dismutase is of animal, human, bacterial, yeast, plant or marine origin or obtained by biotechnology.

6. Composition according to any one of Claims 1 to 5, characterized in that the superoxide dismutase contains iron, contains manganese or contains copper-zinc.

7. Composition according to any one of Claims 1 to 6, characterized in that the polyphosphonic derivative is chosen from ethylenediaminetetra(methylenephosphonic) acid, hexamethylenediaminetetra(methylenephosphonic) acid, diethylenetriaminepenta(methylenephosphonic) acid, 1-hydroxyethylidene-1,1-diphosphonic acid and aminotri(methylenephosphonic) acid and preferably from ethylenediaminetetra(methylenephosphonic) acid and diethylenetriaminepenta(methylenephosphonic) acid, and their salts.

8. Composition according to any one of Claims 1 to 7, characterized in that the salts are chosen from those of ammonium and of alkali metals, preferably of sodium.

9. Composition according to any one of Claims 1 to 8, characterized in that it is in the form of a lotion, an emulsion, a milk, microspheres, microgranules, a cream, a gel, a balm or an aerosol spray, or vesiculas of ionic or non-ionic type.

10. Composition according to any one of Claims 1 to 9, characterized in that it additionally contains at least one cosmetic ingredient chosen from surfactants, dyes, fragrances, preserving agents, emulsifiers, liquid vehicles, fatty substances, resins and waxes.

11. Use of at least one superoxide dismutase in combination with at least one phosphonic derivative, preferably chosen from ethylenediaminetetra(methylenephosphonic) acid, hexamethylenediaminetetra(methylenephosphonic) acid, diethylenetriaminepenta(methylenephosphonic) acid, 1-hydroxyethylidene-1,1-diphosphonic acid and aminotri(methylenephosphonic) acid, and their salts, in the preparation of a cosmetic composition intended for the treatment of the skin.

12. Cosmetic treatment process for the purpose of improving the aesthetic appearance of the skin, characterized in that a cosmetic composition according to Claims 1 to 10 is supplied to the skin.

## Patentansprüche

1. Kosmetische oder pharmazeutische Zubereitung, die zum topischen Gebrauch bestimmt und dadurch gekennzeichnet ist, daß sie mindestens eine Superoxid-Dismutase in Kombination mit mindestens einem Phosphonsäurederivat oder einem seiner Salze umfaßt.

2. Zubereitung nach Anspruch 1, dadurch gekennzeichnet, daß die Superoxid-Dismutase und das Phosphonsäurederivat in einem Gewichtsverhältnis im Bereich zwischen 2 % und 25 % vorhanden sind.

3. Zubereitung nach Anspruch 1 oder 2, dadurch gekennzeichnet, daß die Superoxid-Dismutase in einem Gewichtsverhältnis von 0,001 Gew.-% bis 4 Gew.-%, vorzugsweise von 0,75 Gew.-% bis 1,7 Gew.-%, in bezug auf das Gesamtgewicht der Zusammensetzung vorhanden ist.

4. Zubereitung nach einem der Ansprüche 1 bis 3, dadurch gekennzeichnet, daß das Phosphonsäurederivat in einem Mengenverhältnis von 0,005 Gew.-% bis 2 Gew.-%, vorzugsweise von 0,05 Gew.-% bis 0,1 Gew.-%, in bezug auf das Gesamtgewicht der Zusammensetzung vorhanden ist.

5. Zubereitung nach einem der Ansprüche 1 bis 4, dadurch gekennzeichnet, daß die Superoxid-Dismutase einen tierischen, menschlichen oder bakteriellen Ursprung hat, oder von Hefe oder Pflanzen oder aus dem Meer stammt, oder auch durch Biotechnologie erhalten worden ist.

6. Zubereitung nach einem der Ansprüche 1 bis 5, dadurch gekennzeichnet, daß die Superoxid-Dismutase vom Eisen-, Mangan- oder Kupfer/Zink-Typ (Fe-, Mn- oder $Cu_2Zn_2$-SOD) ist.

7. Zubereitung nach einem der Ansprüche 1 bis 6, dadurch gekennzeichnet, daß das Polyphosphonsäurederivat aus der Ethylendiamintetra-(methylenphosphonsäure), Hexamethylendiamintetra-(methylenphosphonsäure), Diethylentriaminpenta-(methylenphosphonsäure), 1-Hydroxyethyliden-1,1-diphosphonsäure, Aminotri-(methylenphosphonsäure) und vorzugsweise aus der Ethylendiamintetra-(methylenphosphonsäure) und der Diethylentriaminpenta-(methylenphosphonsäure) sowie deren Salzen ausgewählt ist.

8. Zubereitung nach einem der Ansprüche 1 bis 7, dadurch gekennzeichnet, daß die Salze aus den Ammonium- und Alkalimetallsalzen, vorzugsweise dem Natriumsalz ausgewählt sind.

9. Zubereitung nach einem der Ansprüche 1 bis 8, dadurch gekennzeichnet, daß sie in Form einer Lotio, Emulsion oder Milch, in Form von Mikrokügelchen, Mikrogranulatteilchen, Creme, Gel, Salbe oder einer Aerosoldose oder in Form von ionischen oder nichtionischen Vesikeln vorliegt.

10. Zubereitung nach einem der Ansprüche 1 bis 9, dadurch gekennzeichnet, daß sie zusätzlich noch mindestens einen kosmetischen Bestandteil enthält, der aus Tensiden, Farbstoffen, Duftstoffen, Konservierungsmitteln, Emulgatoren, flüssigen Trägern, Fettkörpern, Harzen und Wachsen ausgewählt ist.

11. Verwendung mindestens einer Superoxid-Dismutase in Kombination mit mindestens einem Phosphonsäurederivat, das vorzugsweise aus der Ethylendiamintetra-(methylenphosphonsäure), Hexamethylendiamintetra-(methylenphosphonsäure), Diethylentriaminpenta-(methylenphosphonsäure), 1-Hydroxyethyliden-1,1-diphosphonsäure und Aminotri-(methylenphosphonsäure) sowie deren Salzen bei der Herstellung einer zur Hautbehandlung bestimmten kosmetischen Zubereitung ausgewählt ist.

12. Verfahren zur kosmetischen Behandlung im Hinblick auf die Verbesserung des ästhetischen Aussehens der Haut, dadurch gekennzeichnet, daß auf die Haut eine kosmetische Zubereitung nach den Ansprüchen 1 bis 10 aufgetragen wird.